# EUROPEAN PATENT SPECIFICATION

(11) **EP 3 283 017 B1**
(45) Date of publication and mention of the grant of the patent: **01.01.2020**
(21) Application number: 15721109.5
(22) Date of filing: 17.04.2015
(51) Int. Cl.: A61F 2/42, A61F 2/28

(54) **INBONE TALAR DOME WITH EXPANDABLE FLANGES**
TALUSROLLE IM KNOCHEN MIT EXPANDIERBAREN FLANSCHEN
DÔME ASTRAGALIEN INTRA-OSSEUX COMPORTANT DES PATTES EXTENSIBLES

(43) Date of publication of application: 21.02.2018
(73) Proprietor: Wright Medical Technology, Inc., Memphis, TN 38117 (US)
(72) Inventor: NACHTRAB, Dean, Memphis, TN 38103 (US)
(74) Representative: Gerbino, Angelo
(86) International application number: PCT/US2015/026349
(87) International publication number: WO 2016/167792

(56) References cited:
- WO-A1-2013/153435
- US-A- 6 136 032
- US-A1- 2009 292 364
- US-A1- 2010 161 068
- US-A1- 2011 035 019
- US-A1- 2012 123 553
- US-A1- 2012 245 701
- US-A1- 2014 107 713
- US-B2- 6 613 093

## Description

### BACKGROUND

An ankle joint may become severely damaged and painful due to arthritis, prior ankle surgery, bone fracture, osteoarthritis, and/or one or more additional conditions. Options for treating the injured ankle have included anti-inflammatory and pain medications, braces, physical therapy, joint arthrodesis, and total ankle replacement.

Total ankle replacement generally comprises two components - one component coupled to the tibia and one component coupled to the talus. The components comprise articulation surfaces sized and configured to mimic the range of motion of the ankle joint. For example, the talar portion may comprise a component sized and configured to mimic the talar dome and the tibial portion may comprise an articulation surface configured to mimic articulation of the tibia.

Installation of the total ankle replacement may comprise forming one or more holes or cuts in a bone. For example, a hole may be drilled through the talus and into the tibia to create a channel for inserting a tibial stem. In some installations, additional bone is removed from the talus to make space for a talar stem extending from the talar portion.

US 2012/123553 A1 discloses a joint prosthesis with joint implant components comprising one or more expandable, hollow segmented posts, and having the features of the preamble of claim 1 which follows.

US 2009/292364 A1 discloses a shoulder joint implant assembly including a plastic ball secured to a first joint defining bone end face and a plastic receiver secured to a second joint defining bone end face. Each of the ball and receiver implants are constructed of a composite hardened plastic with a softened cartilage wear surface plastic. A fastener is secured to the first joint defining bone end face and over which is mounted the plastic ball. Anchoring of the receiver is established by a pair of integrally formed and inwardly extending mounting tabs further extending into further recess machined cavities in the first bone.

US 2010/161068 A1 discloses a joint part prosthesis, particularly for a finger joint, consisting either of a proximal or a distal component as well as a shaft extending away from this component, to be mounted in a bone. This shaft is at least partially formed as an expansion sleeve.

### SUMMARY

The present invention is defined in claim 1 and concerns a joint replacement implant. The implant comprises a body comprising a bone contact surface and an articulation surface. The implant further comprises an expandable stem extending longitudinally from the bone contact surface. The expandable stem comprises a plurality of flanges. The plurality of flanges are expandable from a first diameter to a second diameter to anchor the implant to a bone. The second diameter is greater than the first diameter.

In various embodiments, a system is disclosed. The system comprises a tibial implant and a talar implant. The tibial implant comprises a tibial stem and a first joint articulation surface. The talar implant comprises a body defining a second joint articulation surface and a bone contact surface. An expandable stem extends longitudinally from the bone contact surface. The expandable stem comprises a plurality of flanges expandable from a first diameter to a second diameter to anchor the talar implant to a bone. The second diameter is greater than the first diameter.

### BRIEF DESCRIPTION OF THE FIGURES

The features and advantages of the present invention will be more fully disclosed in, or rendered obvious by the following detailed description of the preferred embodiments, which are to be considered together with the accompanying drawings wherein like numbers refer to like parts and further wherein:
FIG. 1 illustrates an anatomic view of an ankle joint.
FIG. 2 illustrates an ankle joint having an embodiment of a total ankle replacement system therein.
FIG. 3 illustrates one embodiment of an implant including a stem having a plurality of expandable flanges.
FIG. 4 illustrates a bottom-view of the implant of FIG. 3.
FIG. 5 illustrates the implant of FIG. 3 coupled to a bone.
FIG. 6 illustrates one embodiment of an implant having a stem including two expandable flanges.
FIG. 7 is a flowchart illustrating one embodiment of a method for coupling an implant having a plurality of expandable flanges to a bone.

### DETAILED DESCRIPTION

The description of the exemplary embodiments is intended to be read in connection with the accompanying drawings, which are to be considered part of the entire written description. In the description, relative terms such as "lower," "upper," "horizontal," "vertical," "proximal," "distal," "above," "below," "up," "down," "top" and "bottom," as well as derivatives thereof (e.g., "horizontally," "downwardly," "upwardly," etc.) should be construed to refer to the orientation as then described or as shown in the drawing under discussion. These relative terms are for convenience of description and do not require that the apparatus be constructed or operated in a particular orientation. Terms concerning attachments, coupling and the like, such as "connected" and "interconnected," refer to a relationship wherein structures are secured or attached to one another either directly or indirectly through intervening structures, as well as both movable or rigid attachments or relationships, unless expressly described otherwise.

The present disclosure generally provides a bone implant for use with a joint replacement system. The bone implant comprises a body having a bone contact surface and an articulation surface. An expandable stem extends longitudinally from the bone contact surface. The expandable stem is sized and configured to be inserted into a hole formed in a bone in an unexpanded state. The expandable stem is expanded into contact with a sidewall of the hole to anchor the implant to the bone.

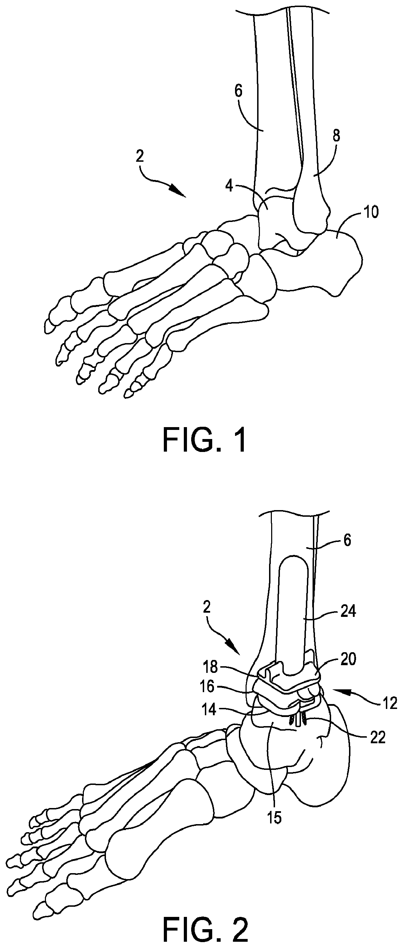

FIG. 1 illustrates an anatomic view of an ankle joint 2. The ankle joint 2 comprises a talus 4 in contact with a tibia 6 and a fibula 8. A calcaneus 10 is located below the talus 4. In total ankle replacements, the talus 4 and the tibia 6 may be resected, or cut, to allow insertion of a talar implant and a tibial implant. FIG. 2 illustrates the ankle joint 2 of FIG. 1 having a total ankle replacement system 12 inserted therein.

The total ankle replacement system 12 comprises a talar platform 14 and a tibial platform 18. The talar platform 14 comprises a body having an integrated articulation surface (or talar dome) and a flange 22. The flange 22 extends into the talus 4 to anchor the talar platform 14 to the talus 4. The tibial platform 18 is sized and configured for installation into the tibia 6. The tibial platform 18 comprises a body 20 coupled to an articulation surface 16 and a tibial stem 24 extending into the tibia 6 to anchor the tibial platform 18. The articulation surface 16 and the talar platform 4 are mutually sized and configured to articulate. The natural ankle joint is mimicked by the interface between the talar body 14 and the articulation surface 16 of the tibial platform. One or more holes may be formed in the tibia and/or the talus prior to and during insertion of the tibial implant 18 or the talar implant 14. For example, in some embodiments, a hole is drilled starting in the bottom of the talus, extending through the talus and into the tibia. The hole may comprise, for example, a 6mm hole configured to guide the reaming for the stem 24 of the tibial platform 18.

The articulation surface 16 and/or the talar body 14 may be made of various materials, such as, for example, polyethylene, high molecular weight polyethylene (HMWPE), rubber, titanium, titanium alloys, chrome cobalt, surgical steel, and/or any other suitable metal, ceramic, sintered glass, artificial bone, and/or any combination thereof. The joint surfaces 16, 20 may comprise different materials. For example, the tibial joint surface 20 may comprise a plastic or other non-metallic material and the talar joint surface 16 may comprise a metal surface. Those skilled in the art will recognize that any suitable combination of materials may be used.

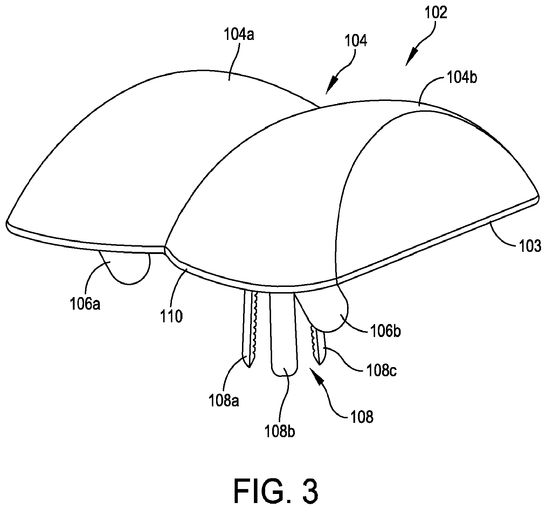

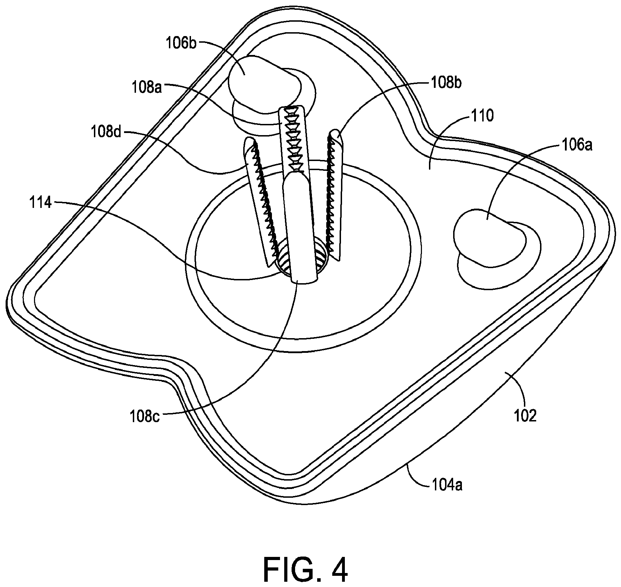

FIGS. 3 and 4 illustrate one embodiment of a talar implant 102. FIG. 3 illustrates a top-side view of the talar implant 102 and FIG. 4 illustrates a bottom-side view of the talar implant 102. The talar implant 102 comprises a body 103 having an articulation surface 104 comprising a first hemisphere 104a and a second hemisphere 104b. The articulation surface 104 is sized and configured to mimic a talar dome and to allow articulation of a tibial implant that is installed opposite the talar implant 102 (see FIG. 2.). The body 103 further comprises a bone contact surface 110 opposite the articulation surface 104. The bone contact surface 110 is sized and configured to contact a talus that has been prepared to receive the talar implant 102, for example, by resectioning.

In some embodiments, the implant 102 comprises one or more positioning stems 106a, 106b extending longitudinally from the bone contact surface. The positioning stems 106a, 106b are configured to position the implant 102 in a proper alignment with respect to a resected talus. The positioning stems 106a, 106b are inserted into one or more holes formed in the talus, for example, during resectioning. The positioning stems 106a, 106b may extend at an angle from the bone contact surface 110. In some embodiments, the positioning stems 106a, 106b extend at an angle between 0-90°, such as, for example, 45°, from the bone contact surface. Although the positioning stems 106a, 106b are illustrated extending at the same angle, it will be recognized that a first positioning stem 106a may extend a first distance at a first angle from the bone contact surface 110 and a second positioning stem 106b may extend a second distance at a second angle from the bone contact surface 110. The positioning stems 106a, 106b prevent rotation of the implant 102 with respect to the bone.

According to the invention, the implant 102 includes an expandable stem 108. The expandable stem 108 has a plurality of flanges 108a-108d. The plurality of flanges 108a-108d are configured to be inserted into a hole formed in a bone, such as a talus. The plurality of flanges 108a-108d are expanded into contact with an inner wall of the hole after inserting the talar implant 102 into the bone. The plurality of flanges 108a-108d may be expanded by any suitable expansion device. For example, in some embodiments, the plurality of flanges 108a-108d are expanded by a screw (not shown) being inserted between the flanges 108a-108d. According to the invention, the flanges 108a-108d comprise partial threading 112 configured to interface with threads of the expansion device. The expansion mechanism drives the plurality of flanges 108a-108d apart and into contact with the sidewall of the hole in the bone.

The flanges 108a-108d are sized and configured to be inserted into a channel formed during preparation of an ankle joint for a total ankle replacement surgery. For example, the flanges 108a-108d are sized and configured to fit within a channel formed in a talus during total ankle replacement surgery. The flanges 108a-108d have an unexpanded diameter smaller than the diameter of the channel and are expandable to a diameter equal to at least the diameter of the channel to anchor the implant 102.

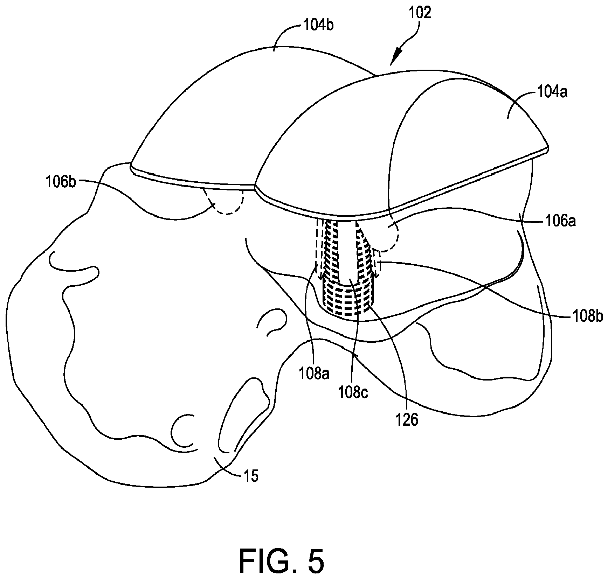

FIG. 5 illustrates one embodiment of the implant 102 coupled to a talus 4. The expandable stem 108 is inserted into a channel 120 formed in the talus 4. The channel 120 is formed in the talus 4, for example, during a resectioning procedure. The channel 120 has a suitable diameter for receiving the expandable stem 108 therein. For example, in one embodiment, the channel has a 6mm diameter. In some embodiments, the channel 120 extends through the tibia 4. The flanges 108a-108d of the expandable stem 108 are inserted through a first side of the talus 4 and into the channel 120. The use of an expandable stem 108 allows a smaller hole to be used as compared to traditional stems which require a larger hole in the talus 4 to receive a talar stem.

After the implant 102 is seated with the stem 108 in the channel 120, the flanges 108a-108d are expanded into contact with an inner surface of the channel 120. n expansion device 126 is inserted between the flanges 108a-108d to expand the flanges 108a-108d into contact with sidewalls of the channel 120. The expansion device 126 is inserted through a second side of the channel 120. The expansion device 126 includes a thread pattern sized and configured to couple to the partial threads 112 formed on the flanges 108a-108d. For example in some embodiments, the expansion device 126 comprises a screw sized and configured to interface with the partial threads 112 formed on the flanges 108a-108d. The bone contact surface 110 may define a screw hole 114 comprising a plurality of threads 116 configured to mate with the threads of the expansion device 126 to couple the expansion device 126 and the implant 102. As the expansion device 126 is driven into contact with the flanges 108a-108d and the threads 116, the expansion device 126 forces the flanges 108a-108d to expand and contact sidewalls of the channel 120. The flanges 108a-108d are expanded into a friction coupling with the sidewalls of the channel 120 and maintain the talar implant 102 in position with respect to the talus 4. In some embodiments, the flanges 108a-108d are used in conjunction with, for example, a cement to maintain the talar implant 102 in contact and alignment with the talus 4.

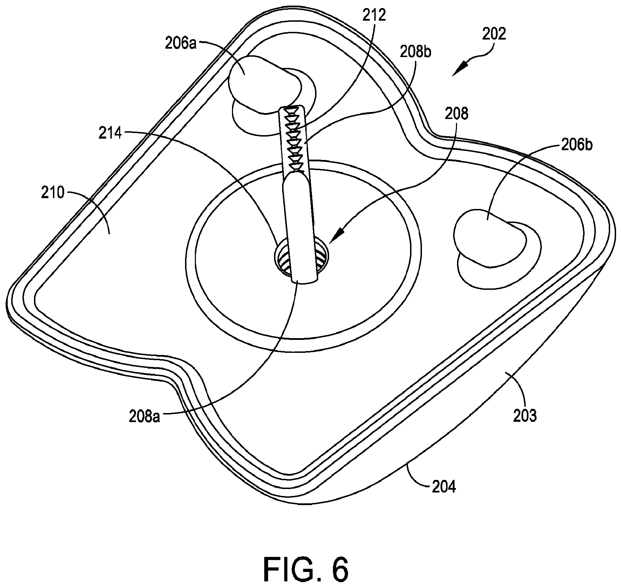

FIG. 6 illustrates one embodiment of an implant 202 having an expandable stem 208 comprising a first flange 208a and a second flange 208b. The implant 202 is similar to the implant 102 discussed with respect to FIGS. 3-5. The implant 202 comprises a body 203 having an articulation surface 204. The articulation surface 204 is sized and configured to mimic a joint surface, such as, for example, talar dome. The body 203 further comprises a bone contact surface 210 opposite the joint surface 204. The bone contact surface 210 is configured to contact a resected bone section. In some embodiments, a plurality of positioning stems 206a, 206b extend from the bone contact surface 210. The plurality of positioning stems 206a, 206b may be received within one or more holes formed in the bone to position the implant 202 in a predetermined location and orientation. The holes may be drilled and/or reamed into the bone.

The implant 202 includes an expandable stem 208. The expandable stem 208 has a first flange 208a and a second flange 208b. The first and second flanges 208a, 208b are configured to be inserted into a hole formed in a bone, such as a talus 4. The first and second flanges 208a, 208b are expanded into contact with an inner wall of the hole after the expandable stem 208 is inserted into the bone. The first and second flanges 208a, 208b can be expanded by any suitable means. For example, in some embodiments, the first and second flanges 208a, 208b are expanded by a screw inserted between the flanges 208a, 208b. The flanges 208a, 208b comprise partial threading 212 configured to interface with the screw. The screw, or other expansion mechanism, drives the flanges 208a, 208b apart and into contact with the sidewall of the hole in the bone. In some embodiments, a threaded hole is formed in the bone contact surface 210 to couple the implant 202 to the screw and maintain the screw in a fixed position. Although embodiments are illustrated showing two and four flanges, it will be recognized that the expandable stems 108, 208 may comprise any number of expandable flanges.

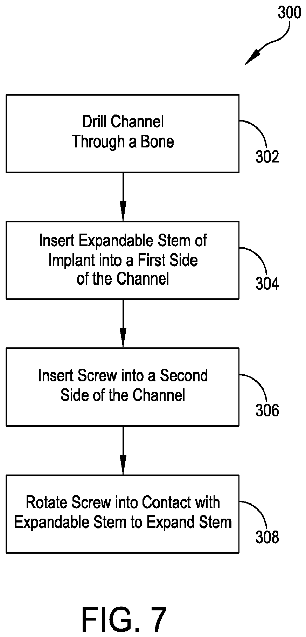

FIG. 7 is a flowchart illustrating one embodiment of a method 300 for installing an implant having an expandable stem. In a first step 302, a channel is formed in a bone. For example, in some embodiments, a channel is formed from a first side of a bone to a second side of a bone. The bone may comprise, for example, a talus. The hole may comprise any suitable diameter, such as, for example, 6mm diameter. In a second step 304, the expandable stem of the implant is inserted into a first side of the channel. The implant may comprise, for example, one of the implants 102, 202 illustrated in FIGS. 1-6. In some embodiments, the implant includes an artificial joint body comprising a bone contact surface and an articulation surface. The expandable stem extends longitudinally from the bone contact surface. The expandable stem includes a plurality of flanges. In some embodiments, the implant is a talar implant sized and configured to mimic a talar dome.

In a third step 306, an expansion device, such as, for example, a screw, is inserted into a second side of the channel formed in the bone. The expansion device is inserted into the channel until the expansion device contacts the expandable flanges of the implant. In a fourth step 308, the expansion device is rotated or otherwise driven into contact with the expandable flanges to expand the expandable flanges into contact with an inner wall of the channel. The expansion device may be driven into a hole formed in the implant. The hole may comprise a coupling mechanism, such as, for example, an internal threading, sized and configured to couple to the expansion device. Although the illustrated embodiment includes a screw, those skilled in the art will recognize that any type of expansion device may be inserted into a second side of the channel formed in the bone.

In various embodiments, an implant is disclosed. The implant comprises a body comprising a bone contact surface and an articulation surface. An expandable stem extends longitudinally from the bone contact surface. The expandable stem comprises a plurality of flanges. The plurality of flanges are expandable from a first diameter to a second diameter. The second diameter is greater than the first diameter.

The plurality of flanges are configured to be expanded by driving an expansion device between the plurality of flanges to expand the plurality of flanges to the second diameter. Each of the plurality of flanges defines a partial threading sized and configured to mate with the expansion device. The body defines a hole having an opening centered between the plurality of expandable flanges and extending from the bone contact surface into the body. The hole comprises a plurality of threads sized and configured to mate with the expansion device. In some embodiments, plurality of expandable flanges comprises four flanges.

In some embodiments, at least one positioning stem extends longitudinally from the bone contact surface at a first angle. The first angle is between 0 and 90 degrees. In some embodiments, the first diameter is less than about 6mm and the second diameter is greater than or equal to about 6mm. The expandable stem extends a longitudinal distance from the bone contact surface, wherein the longitudinal distance is less than or equal to a thickness of the body between the bone contact surface and the articulation surface. In some embodiments, the bone comprises a talus.

In various embodiments, a system is disclosed. The system includes a tibial implant and a talar implant. The tibial implant comprises a tibial stem and a first joint articulation surface. The talar implant comprises a body defining a second joint articulation surface and a bone contact surface. An expandable stem extends longitudinally from the bone contact surface. The expandable stem includes a plurality of flanges expandable from a first diameter to a second diameter. The second diameter is greater than the first diameter.

The system includes an expansion device. The expandable stem is expanded from the first diameter to the second diameter by driving the expansion device between the plurality of flanges. The expansion device may be a screw. Each of the plurality of flanges comprises a partial thread sized and configured to interface with the expansion device.

In some embodiments, the bone contact surface of the talar implant defines a hole centered between the plurality of flanges and extending from the bone contact surface into the body. The hole comprises a thread sized and configured to interface with the expansion device. The expandable stem extends a longitudinal distance less than or equal to a thickness of the body measured between the second joint articulation surface and the bone contact surface.

The invention may be used in a method, which comprises drilling a channel in a bone. The channel extends from a first side of the bone to a second side of the bone. The method further includes inserting an expandable stem of an implant into a first side of the channel. The implant includes an artificial joint body comprising a bone contact surface and an articulation surface. The expandable stem extends longitudinally from the bone contact surface and includes a plurality of flanges. The method further includes expanding the plurality of expandable flanges from a first diameter to a second diameter. The second diameter is greater than the first diameter. The implant is maintained in a fixed position with respect to the bone when the plurality of flanges are expanded to the second diameter.

Expanding the plurality of expandable flanges includes inserting an expansion device into a second side of the channel and driving the expansion between the plurality of flanges to expand the plurality of flanges to the second diameter. In some embodiments, the method further includes driving the expansion device into a hole defined by the body. The hole extends from the bone contact surface into the body. The hole comprises a locking mechanism configured to lock the expansion device in a fixed position.

Although the subject matter has been described in terms of exemplary embodiments, it is not limited thereto. Rather, the invention is defined by the appended claims, which should be construed broadly, to include other variants and embodiments, which may be made by those skilled in the art.

## Claims

1. An implant, comprising:
a body (103) comprising a bone contact surface (110) and an articulation surface (104); and
an expandable stem (108) extending longitudinally from the bone contact surface (110), the expandable stem (108) comprising a plurality of flanges (108a-108d), wherein the plurality of flanges (108a-108d) are expandable from a first diameter to a second diameter, wherein the second diameter is greater than the first diameter, and
wherein the plurality of flanges (108a-108d) are configured to be expanded by driving an expansion device between the plurality of flanges (108a-108d) to expand the plurality of flanges (108a-108d) to the second diameter,
said implant being **characterized in that** each of the plurality of flanges (108a-108d) defines a partial threading sized and configured to mate with the expansion device, wherein the expandable stem (108) is adapted to be inserted through a first side of a channel formed in a bone and the expansion device is adapted to be inserted through a second side of the channel.

2. The implant of claim 1, wherein the body (103) defines a hole having an opening centered between the plurality of expandable flanges (108a-108d) and extending from the bone contact surface (110) into the body (103), the hole comprising a plurality of threads sized and configured to mate with the expansion device.

3. The implant of claim 1, wherein the plurality of expandable flanges (108a-108d) comprises four flanges.

4. The implant of claim 1, further comprising at least one positioning stem (106-a106b) extending longitudinally from the bone contact surface (110) at a first angle.

5. The implant of claim 4, wherein the first angle is between 0 and 90 degrees.

6. The implant of claim 1, wherein the first diameter is less than about 6 mm and the second diameter is greater than or equal to about 6 mm.

7. The implant of claim 1, wherein the expandable stem (108) extends a longitudinal distance from the bone contact surface (110), wherein the longitudinal distance is less than or equal to a thickness of the body (103) between the bone contact surface (110) and the articulation surface (104).

8. The implant of claim 1, wherein the bone comprises a talus.

9. A system, comprising:
a tibial implant (18) comprising a tibial stem (24) and a first joint articulation surface; and
a talar implant (102) according to claim 1, wherein the expansion device comprises a screw.

10. The system of claim 9, wherein the bone contact surface (110) of the talar implant defines a hole centered between the plurality of flanges(108a-108d) and extending from the bone contact surface (110) into the body (103), and wherein the hole comprises a thread sized and configured to interface with the expansion device.

11. The system of claim 9, wherein the expandable stem (108) extends a longitudinal distance less than or equal to a thickness of the body measured between the second joint articulation surface (104) and the bone contact surface (110).

## Patentansprüche

1. Implantat, umfassend;
einen Körper (103), welcher eine Knochenkontaktfläche (110) und eine Bewegungsfläche (104) umfasst; und
einen expandierbaren Schaft (108), welcher in Längsrichtung von der Knochenkontaktfläche (110) ausgeht, wobei der expandierbare Schaft (108) eine Vielzahl von Flanschen (108a-108d) umfasst, wobei die Vielzahl von Flanschen (108a-108d) von einem ersten Durchmesser auf einen zweiten Durchmesser expandierbar sind, wobei der zweite Durchmesser größer als der erste Durchmesser ist, und
wobei die Vielzahl von Flanschen (108a-108d) so konfiguriert sind, dass sie durch Antreiben einer Expansionsvorrichtung zwischen der Vielzahl Flanschen (108a-108d) zum Expandieren der Vielzahl von Flanschen (108a-108d) auf den zweiten Durchmesser expandiert werden,
wobei das Implantat **dadurch gekennzeichnet ist, dass** jeder der Vielzahl von Flanschen (108a-108d) ein Teilgewinde definiert, welches so bemessen und konfiguriert ist, dass es mit der Expansionsvorrichtung zusammenpasst, wobei der expandierbare Schaft (108) dafür ausgebildet ist, durch eine erste Seite eines in einem Knochen gebildeten Kanals eingeführt zu werden und die Expansionsvorrichtung dafür ausgebildet ist durch eine zweite Seite des Kanals eingeführt zu werden.

2. Implantat nach Anspruch 1, wobei der Körper (103) ein Loch definiert, welches eine Öffnung hat, die zwischen der Vielzahl von expandierbaren Flanschen (108a-108d) zentriert ist und von der Knochenkontaktfläche (110) aus in den Körper (103) verläuft, wobei das Loch eine Vielzahl von Gewinden umfasst, welche so bemessen und konfiguriert sind, dass sie mit der Expansionsvorrichtung zusammenpasst.

3. Implantat nach Anspruch 1, wobei die Vielzahl von expandierbaren Flanschen (108a-108d) vier Flansche umfasst.

4. Implantat nach Anspruch 1, ferner umfassend mindestens einen Positionierungsschaft (106a, 106b), welcher von der Knochenkontaktfläche (110) aus in ein einem ersten Winkel in Längsrichtung verläuft.

5. Implantat nach Anspruch 4, wobei der erste Winkel zwischen 0 und 90 Grad liegt.

6. Implantat nach Anspruch 1, wobei der erste Durchmesser kleiner als ungefähr 6 mm ist und der zweiter Durchmesser größer als oder gleich ungefähr 6 mm ist.

7. Implantat nach Anspruch 1, wobei der expandierbare Schaft (108) eine Strecke in Längsrichtung von der Knochenkontaktfläche (110) aus verläuft, wobei die Strecke in Längsrichtung kleiner als oder gleich einer Dicke des Körpers (103) zwischen der Knochenkontaktfläche (110) und der Bewegungsfläche (104) ist.

8. Implantat nach Anspruch 1, wobei der Knochen einen Talus umfasst.

9. System, umfassend,
ein Tibia-Implantat (18), welches einen Tibia-Schaft (24) und eine erste Gelenkbewegungsfläche umfasst; und
ein Talus-Implantat (102) nach Anspruch 1, wobei die Expansionsvorrichtung eine Schraube umfasst.

10. System nach Anspruch 9, wobei die Knochenkontaktfläche (110) des Talus-Implantats ein Loch definiert, welches zwischen der Vielzahl von Flanschen (108a-108d) zentriert ist und von der Knochenkontaktfläche (110) aus in den Körper (103) verläuft, und wobei das Loch ein Gewinde umfasst, welches so bemessen und konfiguriert ist, dass es mit der Expansionsvorrichtung koppelt.

11. System nach Anspruch 9, wobei der expandiere Schaft (108) eine Strecke in Längsrichtung verläuft, die kleiner als eine oder gleich einer Dicke des Körpers ist, die zwischen der zweiten Gelenkbewegungsfläche (104) und der Knochenkontaktfläche (110) gemessenen wird.

## Revendications

1. Implant, comprenant :
un corps (103) comprenant une surface de contact avec l'os (110) et une surface d'articulation (104) ; et
une ailette extensible (108) s'étendant longitudinalement à partir de la surface de contact avec l'os (110), l'ailette extensible (108) comprenant une pluralité de pattes (108a-108d), dans lequel la pluralité de pattes (108a-108d) sont extensibles d'un premier diamètre à un second diamètre, dans lequel le second diamètre est plus grand que le premier diamètre, et
dans lequel la pluralité de pattes (108a-108d) sont configurées pour être étendues en amenant un dispositif d'extension entre la pluralité de pattes (108a-108d) pour étendre la pluralité de pattes (108a-108d) au second diamètre,
ledit implant étant **caractérisé en ce que** chacune de la pluralité de pattes (108a-108d) définit un filetage partiel dimensionné et configuré pour s'apparier avec le dispositif d'extension, dans lequel l'ailette extensible (108) est adaptée pour être insérée à travers un premier côté d'un canal formé dans un os et le dispositif d'extension est adapté pour être inséré à travers un second côté du canal.

2. Implant selon la revendication 1, dans lequel le corps (103) définit un trou ayant une ouverture centrée entre la pluralité de pattes extensibles (108a-108d) et s'étendant à partir de la surface de contact avec l'os (110) jusque dans le corps (103), le trou comprenant une pluralité de filetages dimensionnés et configurés pour s'apparier avec le dispositif d'extension.

3. Implant selon la revendication 1, dans lequel la pluralité de pattes extensibles (108a-108d) comprend quatre pattes.

4. Implant selon la revendication 1, comprenant en outre au moins une ailette de positionnement (106a-106b) s'étendant longitudinalement à partir de la surface de contact avec l'os (110) avec un premier angle.

5. Implant selon la revendication 4, dans lequel le premier angle est entre 0 et 90 degrés.

6. Implant selon la revendication 1, dans lequel le premier diamètre est inférieur à environ 6 mm et le second diamètre est supérieur ou égal à environ 6 mm.

7. Implant selon la revendication 1, dans lequel l'ailette extensible (108) s'étend sur une distance longitudinale à partir de la surface de contact avec l'os (110), dans lequel la distance longitudinale est inférieure ou égale à une épaisseur du corps (103) entre la surface de contact avec l'os (110) et la surface d'articulation (104).

8. Implant selon la revendication 1, dans lequel l'os comprend un talus.

9. Système, comprenant :
un implant tibial (18) comprenant une ailette tibiale (24) et une première surface d'articulation de jointure ; et
un implant talien (102) selon la revendication 1, dans lequel le dispositif d'extension comprend une vis.

10. Système selon la revendication 9, dans lequel la surface de contact avec l'os (110) de l'implant talien définit un trou centré entre la pluralité de pattes (108a-108d) et s'étendant à partir de la surface de contact avec l'os (110) jusque dans le corps (103), et dans lequel le trou comprend un filetage dimensionné et configuré pour former une interface avec le dispositif d'extension.

11. Système selon la revendication 9, dans lequel l'ailette extensible (108) s'étend sur une distance longitudinale inférieure ou égale à une épaisseur du corps mesurée entre la seconde surface d'articulation de jointure (104) et la surface de contact avec l'os (110).
